Europäisches Patentamt

European Patent Office

Office européen des brevets

(19)

(11) EP 1 459 682 A1

(12) **EUROPEAN PATENT APPLICATION**

(43) Date of publication:
**22.09.2004 Bulletin 2004/39**

(51) Int Cl.$^7$: **A61B 5/103**

(21) Application number: **04006186.3**

(22) Date of filing: **16.03.2004**

(84) Designated Contracting States:
**AT BE BG CH CY CZ DE DK EE ES FI FR GB GR HU IE IT LI LU MC NL PL PT RO SE SI SK TR**
Designated Extension States:
**AL LT LV MK**

(30) Priority: **20.03.2003 IT MI20030541**

(71) Applicants:
• **ISTITUTO NAZIONALE PER LO STUDIO E LA CURA DEI TUMORI**
  **I-20133 Milano (IT)**
• **MHT S.r.l.**
  **37129 Verona (IT)**

(72) Inventors:
• **Marchesini, Renato Angelo**
  **20133 Milano (IT)**
• **Tomatis, Stefano Maria**
  **20133 Milano (IT)**
• **Carrara, Mauro**
  **20133 Milano (IT)**
• **Berner, Markus**
  **8155 Niederhasli (CH)**

(74) Representative: **Banfi, Paolo**
  **Bianchetti Bracco Minoja S.r.l.**
  **Via Plinio, 63**
  **20129 Milano (IT)**

(54) **Apparatus for the characterisation of pigmented skin lesions**

(57) An apparatus is described, for the characterisation of pigmented skin lesions, which comprises an instrument for the acquisition of a plurality of images of the lesion, filmed with lighting at different wavelengths, means designed to segment and parameterise each of said images, means designed to extrapolate a data set from said images and input said data into a neural network system, means designed to compare the results processed by said neural network with the results obtained following similar processing of known cases, and means designed to vary the weighting of each parameter supplied to the neural network on the basis of said results.

Figure 3

EP 1 459 682 A1

**Description**

This invention relates to an apparatus for the characterisation of pigmented skin lesions which is designed to assist doctors in diagnosing pigmented skin lesions in general, and melanomas and the like in particular.

The apparatus comprises an instrument for the acquisition of a plurality of images of the lesions, filmed with lighting at different wavelengths, means designed to segment and parameterise each of said images, means designed to extrapolate a data set from said images and input said data into a neural network system, means designed to compare the results processed by said neural network with the results obtained following similar processing of known cases, and means designed to vary the weighting of each parameter supplied to the neural network on the basis of said results.

The apparatus then supplies a parameter which allows the lesion to be classified under one of the categories commonly used in clinical diagnosis, such as "probable melanoma", "suspect case", "doubtful case" and "probable non-melanoma".

The importance of early diagnosis of tumours, including epidermal tumours, is well known.

In the specific case of epidermal tumours such as melanomas, early diagnosis is based on visual observation of a series of characteristics of the lesion, among which Asymmetry of the lesion, ragged Border, Colour and Dimension (A B C D) have acquired particular importance over the years.

However, these parameters obviously require subjective evaluation by a doctor, which means that the result is strongly influenced by the doctor's skill and experience, and by incidental factors such as lighting conditions and the like.

Modern technology provides some sophisticated instrumentation which allows enlarged images of the lesion to be obtained under different conditions, but the evaluation and consequent classification of the lesion still depend on the doctor's experience, and his evaluation relies on a visual impression which can vary according to the conditions of the moment.

The modern systems available include epiluminescence microscopy, spectrophotometry methods and infra-red imaging.

The characteristics of the lesion, such as ragged edges, colour and/or presence of darker areas, etc., are parameters that vary to a greater or lesser extent in the presence of disease, enabling the doctor to assess whether or not the lesion belongs to the melanoma category.

The digital imaging technique with evaluation of reflectance under different lighting conditions allows determination of various parameters that may be typical of melanoma, such as cutaneous blood, pigmentation and the presence of melanin, which present different optical characteristics in the presence of disease.

Recent computerised image-processing techniques reveal the morphological characteristics of the lesion and allow study of its structure, the conformation of the vascular network and the presence of any cell aggregates, all of which parameters are very important for the purpose of establishing the existence of a melanoma.

Nevertheless, however useful these systems may be, the assistance they provide is limited, because they are unable to improve the doctor's evaluation skills or enable him to work under standardised conditions, with the result that the doctor's subjective evaluation is still based on his personal experience, and early diagnosis of melanoma still presents a high error rate, even when performed by skilled doctors.

The present invention, which falls into this sector, relates to an apparatus for the characterisation of pigmented skin lesions which is designed to provide doctors with information useful in classifying the lesion by assigning it to a type or group of types including, for example, probable non-melanoma, doubtful cases and probable melanoma.

The apparatus according to the invention is based on the use of a neural network system.

The apparatus comprises an instrument designed to acquire a set of images of the lesion, filmed with lighting at different wavelengths, and to process said images to extract the descriptors of the lesion, means designed to reduce them to a limited number of descriptors, but in such a way as to maintain the total data variance almost entirely, means designed to compare said data with a previously stored data set relating to analysis of a series of lesions in order to extrapolate a value indicative of a type of pathological state, and means designed to recalibrate the system at intervals.

In order to explain the invention more clearly, the apparatus according to the invention and its method of operation will now be described by reference to the annexed drawings, wherein:

fig. 1 is a block diagram of the system of acquisition of digital images with the apparatus according to the invention;

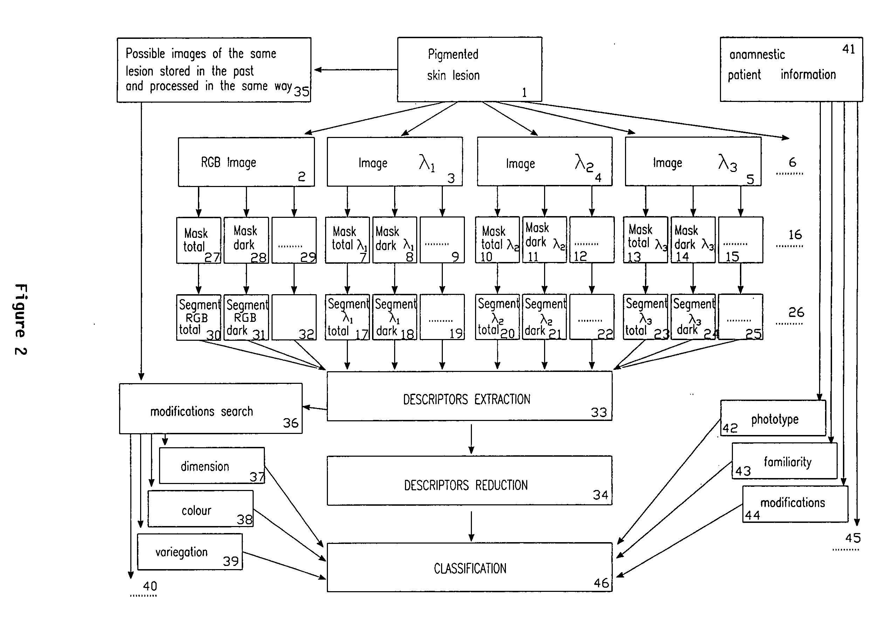

fig. 2 is a block diagram of the method of operation of the apparatus for processing and classification of pigmented skin lesions;

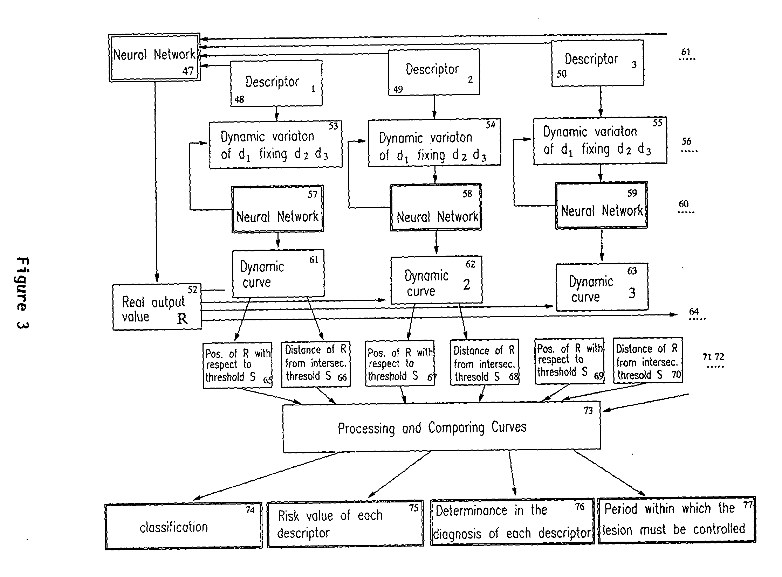

fig. 3 is a schematic representation of the model of dynamic image analysis performed with the apparatus according to the invention;
fig. 4 schematically illustrates an apparatus according to the invention;
fig. 5 is a diagram of the neural network also representing input values $i_1, i_2..., i_6$, the same values $i'_1, i'_2..., i'_6$

normalised and output value *n*; the threshold value is *s=0.7760;*

fig. 6 shows a dynamic lesion-classification curve. Dynamic curve obtained by varying i'$_1$ between 0 and 1. The values on the x-axis are multiplied by 100;

fig. 7a show dynamic curve obtained by varying $i'_1$;

fig. 7b show dynamic curve obtained by varying $i'_2$;

fig. 7c show dynamic curve obtained by varying $i'_3$;

fig. 7d show dynamic curve obtained by varying $i'_4$;

fig. 7e show dynamic curve obtained by varying $i'_5$;

fig. 7f show dynamic curve obtained by varying $i'_6$;

fig. 8 is a histogram representing risk values of lesion examined;

fig. 9 is an example of dynamic curve for a lesion classified as a melanoma; *A(NM)* and *A(CM)* represent the areas under the dynamic curve and the horizontal line corresponding to threshold value s of the network;

fig. 10a show dynamic curve obtained by varying $i'_1$;

fig. 10b show dynamic curve obtained by varying $i'_2$;

fig. 10c show dynamic curve obtained by varying $i'_3$;

fig. 10d show dynamic curve obtained by varying $i'_4$;

fig. 10e show dynamic curve obtained by varying $i'_5$;

fig.10f show dynamic curve obtained by varying $i'_6$;

fig. 11 shows an example of distribution of determinance for the various descriptors of the lesion examined. $p_{inc}(1)=0.91$, $p_{inc}(3)=0.85$, $p_{inc}(6)=0.83$ (see figures 3a,c,f).

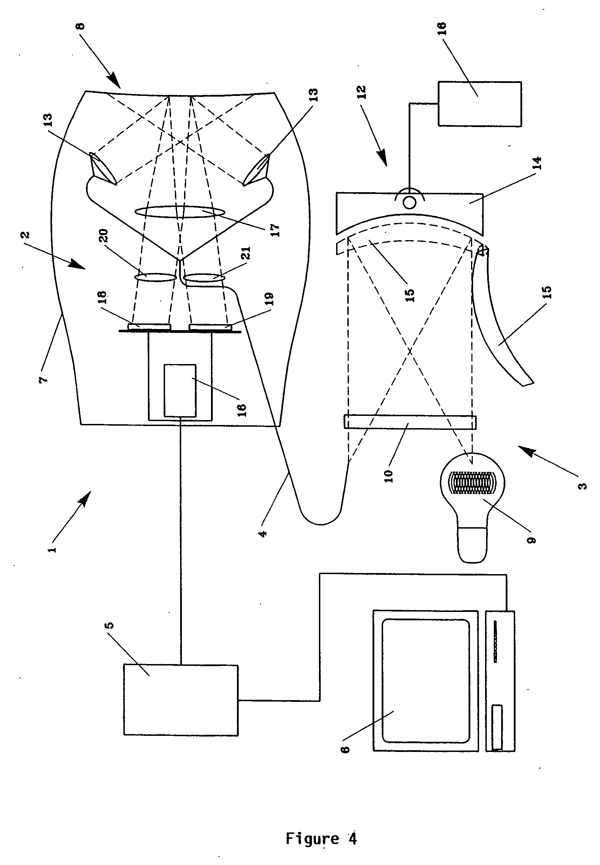

As shown in figure 4, the apparatus according to the invention comprises a probe 1 equipped with an imaging system 2 designed to film the lesion, which is illuminated by a device 3 more particularly described below, said device being connected to probe 1 via a fibre optic cable 4. The probe is then connected via an interface 5 to a computer 6, which in turn is implemented with a neural network.

The neural network may consist of hardware devices or programs in which a set of elements initially has a random connection or a connection entered on the basis of pre-set criteria. The neural network is then taught to recognise a configuration by strengthening the signals that lead to the correct result and weakening incorrect or inefficient signals; the neural network consequently "remembers" this configuration and applies it when processing new data, thus giving rise to a kind of self-learning process.

Probe 1 is installed in a body 7 with an aperture 8 shaped so that it can be rested on the patient's skin around the lesion to be tested.

The lighting device comprises a light source 9, a filter 10 designed to eliminate blue and ultra-violet light, a concave mirror 15, a colour separator 12 and an optical unit 13 for uniform distribution of light in the area of the lesion to be filmed.

Lamp 9 may be a halogen lamp or a xenon lamp, for example.

Acquisition window 8 of the probe may be fitted with a number of legs to adapt it better to the surface of the area studied and allow the probe to be positioned, preferably at right angles to the skin surface, in such a way as to ensure better acquisition of the image by the systems with which the probe is fitted.

Colour separator 12 comprises a step motor 16 which causes a concave mirror 14 with a diffraction grid to rotate around its own axis.

A second concave mirror 15, with no diffraction grid, is fitted between filter 10 and mirror 14 and can be rotated, by means of devices not illustrated in the figure, between a position shown in the figure with a broken line, in which it receives and reflects the illumination, and a position represented by an unbroken line in which it does not interfere with the path of the light.

Mirror with diffraction grid 14 breaks down the light from lamp 9 into a series of spectral bands with pre-selected wavelengths $\lambda_1, \lambda_2, \lambda_3$ ........

Mirror 15 is moved to a position in which it acquires a colour image of the same area by means of a video camera 16, preferably the triple-sensor type, fitted with a lens 17.

Fibre optic bundle 4, which directs the light towards the area to be filmed, is given a ring configuration close to the terminal end, so that the fibres are arranged all round lens 17 of the video camera and illuminate the filming area as uniformly as possible.

A set of optical units 13 serves to distribute the light better.

The imaging system comprises the video camera with a sensor 18 for black and white filming, which is sensitive to infrared rays, and a second sensor, or preferably a set of 3 sensors, 19, for RGB filming.

The optical unit consisting of the lens is schematically represented by lens 17 and two more lenses 20 and 21; however, this is merely a schematic layout, and the optical unit could also be a complex type.

Sensors 18 and 19, preferably constituted by CCD sensors, are both connected to interface 5 and, via said interface, to computer 6.

The assembly will also advantageously comprise a calibrated light source, not illustrated in the figure, for calibration of the device, and in particular for the "blank calibration" to be performed before each set of images is filmed, especially before RGB filming.

To film a lesion, the device is first calibrated by reading a surface lit with a calibrated light, so that the electronics of the device calibrate the reading curves of the sensors in accordance with a known technology.

The apparatus is now ready, and images of the lesion can be obtained by resting the probe on the epidermis in such a way that the lesion is enclosed within reading window 8.

Black and white readings are obtained by removing mirror 15 so that the light from lamp 9 is reflected by mirror with diffraction grid 14.

Mirror 14 is rotated by motor 16 through the angle required to reflect light with the pre-selected wavelength; said light passes through fibre optic cable 14 and illuminates the area to be filmed.

The image is filmed by lens 17, which transfers it to sensor 18; from there, it is conveyed via interface 5 to computer 6 for saving and subsequent processing.

A set of images are filmed, the angle of mirror with diffraction grid 14 being varied each time so as to vary the wavelength of the light that illuminates the lesion.

In this specific case, the device will advantageously be designed to film with light at a wavelength of between 480 and 1000 nanometres.

Mirror 15 is then rotated to reflect all the light from lamp 9, without limiting the wavelength band, in order to perform RGB filming with the second sensor 19.

For the sake of completeness, the use of the apparatus according to the invention for characterisation of a pigmented skin lesion in the diagnosis of melanoma will now be described, with examples.

A set of preliminary instructions is first supplied to the neural network, for example by storing data already acquired in relation to a number of lesions with known histological results.

Information relating, for example, to dimensions, ragged edges, colour of the lesion, etc., is stored, and on the basis of this information the machine performs a first classification of new lesions using an algorithm implemented on the basis of said known data.

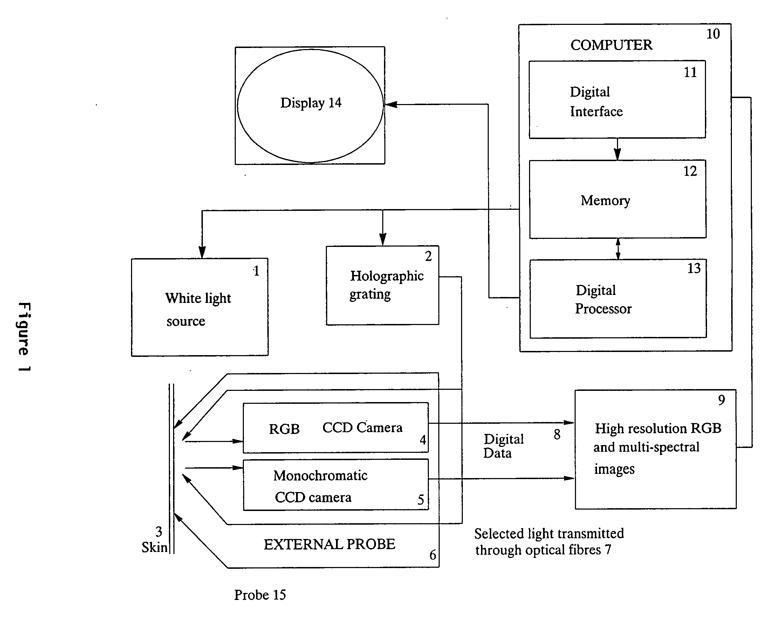

The operation of the apparatus for objective characterisation of a new lesion is represented schematically in the block diagram in Fig. 1.

When the instrument has been calibrated, the probe is placed on the lesion and a digital image thereof acquired.

For this purpose, a first acquisition is performed in RGB format, after which a number of images (in this specific case 15) of the same lesion, illuminated by light with different wavelengths determined by suitable rotation of mirror with diffraction grid 14, are successively acquired and recorded.

Imaging is performed in the field of visible and infra-red radiation, with separate processing of each image.

For each black and white image, a set of parameters which are considered significant for the purpose of determining whether the lesion is a melanoma are obtained.

For example, the most commonly used clinical criterion, known as "A B C D", can be used.

A set of parameters, including size, variegation, reflectance in the visible light zone, infra-red reflectance, the presence of dark patches and the ratio between the area of the dark patches and the rest of the lesion are obtained from each image.

Numerous variables are thus obtained, the number of which is reduced by suitable statistical analysis such as factorial analysis; a limited number of variables can thus be selected, e.g. three for each descriptor, which still retain over 85%, and preferably at least 95% of the variance. These are the variables which will be input into the neural network.

Basically, a set of descriptors of each lesion will be extracted after processing of the images, and successively reduced to the number of six: $\{i_1, i_2, ..., i_6\}$. These six descriptors, when input into the neural network, allow the lesion to be classified. This is done following a comparison between the result $n$ output by the network and the classification threshold value $s$ previously obtained by teaching the neural network and determining the connection weightings between the neurones.

The values of descriptors $i_1, i_2, ..., i_6$ are re-expressed in terms between 0 and 1 in accordance with the following linear normalisation procedure.

The minimum value and maximum value of lesions $m=1,2,3,...$ previously acquired are selected for each descriptor $i_1(1), i_1(2), i_1(3),...$:

$$i_{1,min} = \min_m\left(\{i_1(m)\}\right) \qquad i_{1,max} = \max_m\left(\{i_1(m)\}\right)$$

$$i_{2,min} = \min_m\left(\{i_2(m)\}\right) \qquad i_{2,max} = \max_m\left(\{i_2(m)\}\right)$$

$$i_{3,min} = \min_m\left(\{i_3(m)\}\right) \qquad i_{3,max} = \max_m\left(\{i_3(m)\}\right)$$

$$i_{4,min} = \min_m\left(\{i_4(m)\}\right) \qquad i_{4,max} = \max_m\left(\{i_4(m)\}\right)$$

$$i_{5,min} = \min_m\left(\{i_5(m)\}\right) \qquad i_{5,max} = \max_m\left(\{i_5(m)\}\right)$$

$$i_{6,min} = \min_m\left(\{i_6(m)\}\right) \qquad i_{6,max} = \max_m\left(\{i_6(m)\}\right)$$

Example: if 100 different lesions measuring between $10mm^2$ and $150mm^2$ have been acquired, and $i_6$ is the descriptor relating to the dimensions of the lesion, then $i_{6,min} = 10$ and $i_{6,max} = 150$.

Each value of descriptors $i_1$, $i_2$, ..., $i_6$ is then converted into new values $i'_1$, $i'_2$, ..., $i'_6$ in accordance with the linear equations:

$$i'_1(m) = \frac{i_1(m)}{i_{1,max} - i_{1,min}} + \frac{i_{1,min}}{i_{1,min} - i_{1,max}}$$

$$i'_2(m) = \frac{i_2(m)}{i_{2,max} - i_{2,min}} + \frac{i_{2,min}}{i_{2,min} - i_{2,max}}$$

$$i'_3(m) = \frac{i_3(m)}{i_{3,max} - i_{3,min}} + \frac{i_{3,min}}{i_{3,min} - i_{3,max}}$$

$$i'_4(m) = \frac{i_4(m)}{i_{4,max} - i_{4,min}} + \frac{i_{4,min}}{i_{4,min} - i_{4,max}}$$

$$i'_5(m) = \frac{i_5(m)}{i_{5,max} - i_{5,min}} + \frac{i_{5,min}}{i_{5,min} - i_{5,max}}$$

$$i'_6(m) = \frac{i_6(m)}{i_{6,max} - i_{6,min}} + \frac{i_{6,min}}{i_{6,min} - i_{6,max}}$$

It will immediately be seen that $i'_{1,max}=1$; $i'_{1,min}=0$, $i'_{2,max}=1$; $i'_{2,min}=0,...$ .

Example: all the dimension values cited in the previous example are re-expressed in accordance with the equation:

$$i'_6(m) \quad \frac{i_6(m)}{140} - \frac{1}{14}$$

from which it will immediately be seen that $i'_{6,max}=1$ and $i'_{6,min}=0$; all dimension values between the minimum and maximum values are re-expressed in the interval between 0 and 1.

*A data archive* containing the values of the normalised descriptors $\{i'_1, i'_2, ..., i'_6\}$ relating to all the lesion images already acquired, the colour image *I* of each lesion and the corresponding histological classification *h* (NM: non-melanoma or CM: melanoma) is thus generated and stored in the memory.

| $m=1$ | $i'_1(1)$ | $i'_2(1)$ | $i'_3(1)$ | $i'_4(1)$ | $i'_5(1)$ | $i'_6(1)$ | $I(1)$ | $h(1)$ |
|---|---|---|---|---|---|---|---|---|
| $m=2$ | $i'_1(2)$ | $i'_2(2)$ | $i'_3(2)$ | $i'_4(2)$ | $i'_5(2)$ | $i'_6(2)$ | $I(2)$ | $h(2)$ |
| $m=3$ | $i'_1(3)$ | $i'_2(3)$ | $i'_3(3)$ | $i'_4(3)$ | $i'_5(3)$ | $i'_6(3)$ | $I(3)$ | $h(3)$ |
| ... | ... | ... | ... | ... | ... | ... | ... | ... |
| ... | ... | ... | ... | ... | ... | ... | ... | ... |

Example: for the lesion m=25, the data archive will contain:

| | $i'_1(25)$ | $i'_2(25)$ | $i'_3(25)$ | $I'_4(25)$ | $i'_5(25)$ | $i'_6(25)$ | | |
|---|---|---|---|---|---|---|---|---|
| $m=25$ | 0.3285 | 0.2041 | 0.7460 | 0.4849 | 0.5364 | 0.3275 | $I(25)$: *Image of lesion* | $h=CM$ |

Dynamic analysis of lesions

*A dynamic lesion analysis* is therefore conducted to evaluate the risk level of each descriptor processed, ie. the extent to which a variation in each descriptor risks varying the classification of a lesion classed as a non-melanoma. If the lesion is classified as a melanoma, dynamic analysis evaluates the extent to which each of the descriptors determines that classification. Dynamic lesion analysis also allows lesions to be classified in two or more classes (in this specific case there are four classes: "probable melanoma", "doubtful", "suspect" and "probable non-melanoma").

*Example of processing of a benign lesion*

Dynamic lesion analysis is based on the following principle: five of the six values of the descriptors examined remain fixed in turn, while the sixth value is varied between the minimum and maximum values in the data archive ($i'_{min}=0$ and $i'_{max}=1$ for each descriptor), and the response $n$ which the neural network would give for each value of the sixth simulated descriptor is evaluated.

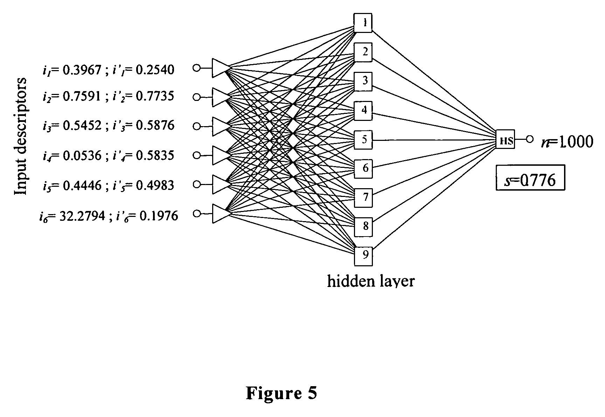

Figure 5 shows the set of values $\{i_1, i_2,..., i_6\}$ acquired by the six different descriptors, which in this specific case are:

$i_1$: presence of dark sub-zones
$i_2$: ratio between areas of dark zones and total area of lesion
$i_3$: variegation
$i_4$: infra-red reflectance
$i_5$: reflectance in the red light zone
$i_6$: size of lesion.

The quantities $i'_1, i'_2, ..., i'_6$ are generated following normalisation of $i_1, i_2, ..., i_6$; $n$ is the value output by the neural network, and $s$ is the classification threshold value with which it is compared.

The diagram of the neural network shown in figure 5 also shows input values $i_1, i_2, ..., i_6$, the same values $i'_1, i'_2, ..., i'_6$ normalised, and output value $n$; the threshold value is $s=0.7760$.

Dynamic analysis simulating the first descriptor

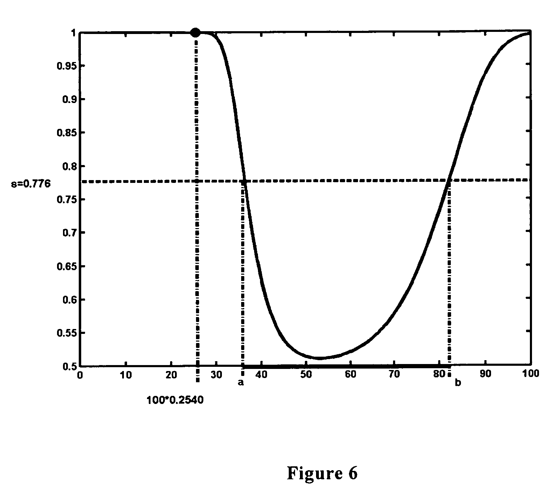

If descriptors $i'_2, i'_3, i'_4, i'_5$ and $i'_6$ are maintained at fixed values, it will be possible to see how value $n$ varies if $i'_1$ acquires another value. Specifically, 1000 $i'_{1,simul}$ values equidistant from one another, falling between 0 and 1, are generated; each set $(i_{1,simul}, i_2, i_3, i_4, i_5, i_6)$ is input into the neural network and generates an output value n. A curve of the type shown in figure 6, called a *dynamic curve,* is thus obtained, in which the values on the x-axis are multiplied by 100 and each point is generated by a different value of $_{1,simul}$ (curve A). Circle C indicates the point corresponding to $i'_1=0.2540$, the true value of the first descriptor of the lesion. As quantity $n$ associated with $i'_1$ is equal to 1 and *s=0.7760,* the lesion is classified as a non-melanoma, because *n>s.*

It will immediately be seen from the graph that if $i'_1$ were between threshold *a* [from *n>s* to *n<s]* and threshold *b* [from *n<s* to *n>s]* (zone a-b), the value of n would be less than threshold value s and the lesion would be classified as a melanoma.

Dynamic analysis simulating the other descriptors

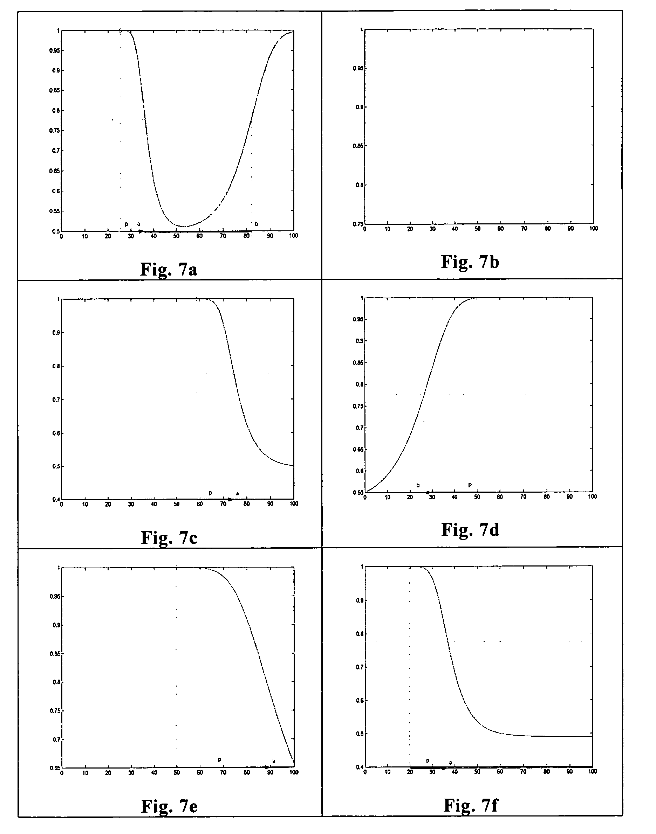

The process illustrated above is also applied to the other five descriptors of the lesions, and a different dynamic curve is obtained for each simulated descriptor. An example of these curves is shown in figures 7a to 7f.

Definition of risk and risk histogram

Two quantities, known as the *index of risk in the event of increase in a descriptor* and *index of risk in the event of decrease in a descriptor,* are defined on the basis of the dynamic curves obtained. These quantities indicate the extent to which a variation in the descriptor in question can cause a change in the initial assessment of non-malignancy of the lesion.

*Risk of increase $p_{inc}$ in descriptor d* (where *d* in this specific case ranges between 1 and 6) is defined as:

$$\begin{cases} p_{inc}(d) = 1 - \left| i'_d - a \right| & \text{wherein } i'_d < a \text{ and } a \text{ is the closest value of the threshold between } n>s \text{ and } n<s \\ p_{inc}(d) = 0 & \text{if } i'_d > a \text{ or } a \text{ is non-existent} \end{cases}$$

and *risk of decrease $p_{dec}$ in descriptor d* is defined as:

$$\begin{cases} p_{dec}(d) = 1 - (i'_d - b) & \text{wherein } i'_d > b \text{ and } b \text{ is the closest value of the threshold between } n<s \text{ and } n>s \\ p_{dec}(d) = 0 & \text{if } i'_d < b \text{ or } b \text{ is non-existent} \end{cases}$$

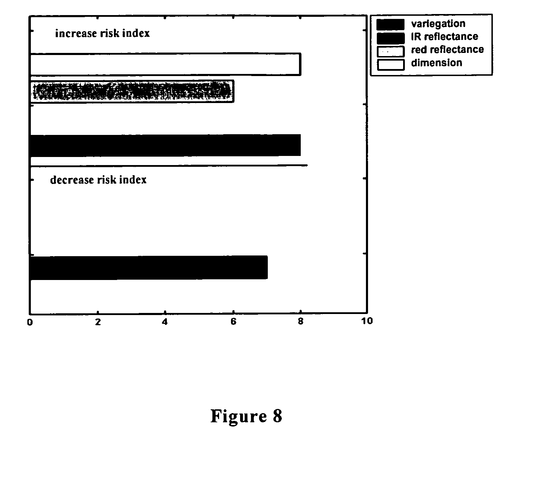

As will be seen from the definitions, the shorter the distance (arrow p in figures 8a, b, c, d, e and f) between the value $i'_d$ and one of the thresholds *a* or *b,* the greater the risk value associated with that descriptor. The risk values obtained can all be summarised in the histogram shown in figure 8, in which the values of *p* are re-expressed in accordance with the following simple formulas in order to assign a discrete risk value between 0 and 10 to each descriptor:

$$p_{inc}^{*}(d) = \text{int}[10 \cdot p_{inc}(d)]$$

$$p_{dec}^{*}(d) = \text{int}[10 \cdot p_{dec}(d)]$$

wherein "int" means the integer of the result obtained in the square brackets.

The evaluation of the risk of variation in the descriptors is of particular clinical interest, as it tells both doctor and patient which characteristics of the lesion must be most closely monitored, and the extent to which they represent a risk.

Example of processing of a melanoma

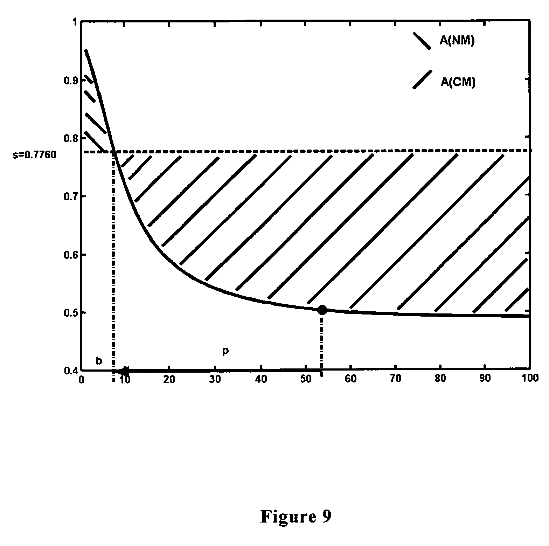

The procedure used to generate dynamic curves is the same in the case of lesions classified as melanomas. However, the information obtained from these curves is different, because the definition of risk obviously does not make sense for lesions classified as melanomas. Figure 9 contains an example of a dynamic curve for a lesion classified as a melanoma; in that figure, *A(NM)* and *A(CM)* represent the areas under the dynamic curve and the horizontal line corresponding to the threshold value s of the network.

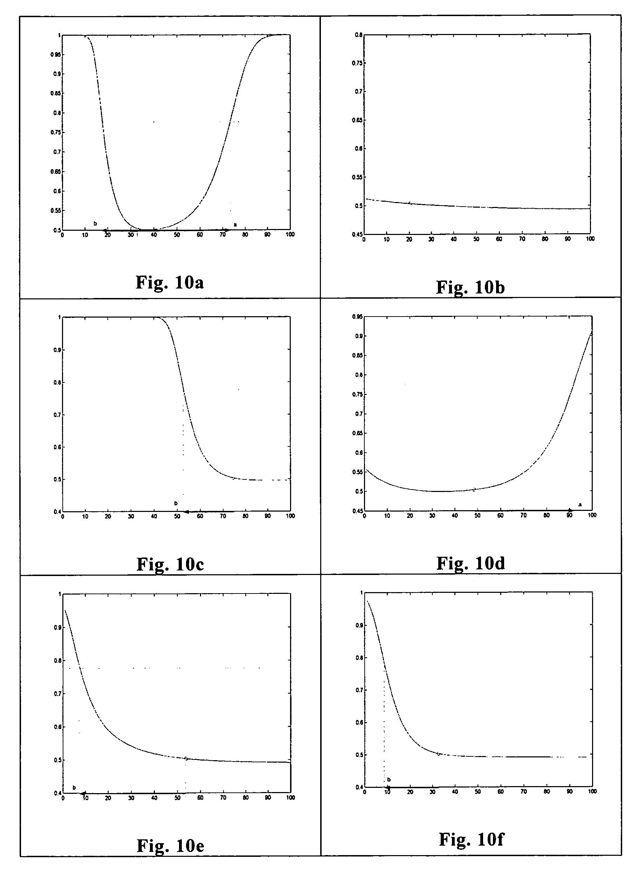

Six sample dynamic curves relating to the various descriptors are shown in figures 10 a, b, c, d, e and f.

Definition of determinance and distribution of determinances

In the case of a lesion classified as a melanoma, reference will therefore not be made to risk, but to the *determinance* $\delta(d)$ of each descriptor. Determinance indicates the extent to which the values acquired by each descriptor d determine the classification of malignancy of the lesion in question. This is expressed for each descriptor by

$$\delta(d) = \delta_{inc}(d)\,) + \delta_{dec}(d)$$

wherein $\delta_{inc}(d)$ and $\delta_{dec}(d)$, called *determinance of increase in descriptor d* and *determinance of decrease in descriptor d* respectively, are defined as follows:

*determinance of increase in descriptor d* (wherein *d* in this specific case ranges between 1 and 6):

$$\begin{cases} \delta_{inc}(d) = \dfrac{A(NM)}{A(CM)} \dfrac{1}{|i'_d - a|} & \text{wherein } i'_d < a \text{ and } a \text{ is the closest threshold value} \\[2em] & \text{from } n<s \text{ to } n>s \\[1em] \delta_{inc}(d) = 0 & \text{if } i'_d > a \text{ or } a \text{ is non-existent} \end{cases}$$

*determinance of decrease in descriptor d:*

$$\begin{cases} \delta_{dec}(d) = \dfrac{A(NM)}{A(CM)} \dfrac{1}{(i'_d - b)} & \text{wherein } i'_d > b \text{ and } b \text{ is the closest threshold value} \\[2em] & \text{from } n>s \text{ to } n<s \\[1em] \delta_{dec}(d) = 0 & \text{if } i'_d < b \text{ or } b \text{ is non-existent} \end{cases}$$

As will be seen, the shorter the distances between $i'_d$ and any thresholds *a* and *b* and the higher the ratio between areas *A(NM)* and *A(CM),* the greater the determinance value δ.

If

$$D = \sum_{d=1}^{6} \delta_{inc}(d) + \sum_{d=1}^{6} \delta_{dec}(d)$$

the components of determinance $\delta_{inc}$ and $\delta_{dec}$ can be re-expressed by

$$\delta_{inc}^{*}(d) = \frac{\delta_{inc}(d)}{D}$$

$$\delta_{dec}^{*}(d) = \frac{\delta_{dec}(d)}{D}$$

to obtain *normalised* determinance value δ*

$$\sum_{d=1}^{6} \delta^{\bullet}(d) = \sum_{d=1}^{6} \delta^{\bullet}_{inc}(d) + \sum_{d=1}^{6} \delta^{\bullet}_{dec}(d) = 1$$

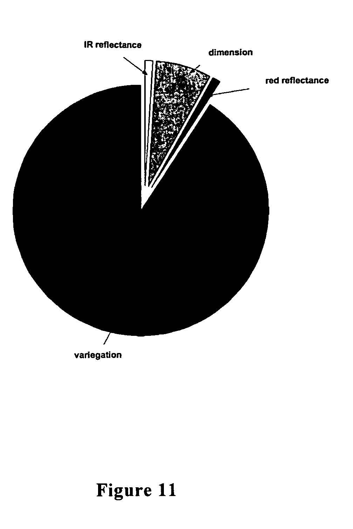

and the distribution of these values can be displayed in the chart contained in figure 11, which shows an example of distribution of determinance for the various descriptors of the lesion in question.

In addition to information about determinance, it is equally important from the clinical standpoint to assess *what* percentage variation in each descriptor would lead to a different classification of the lesion. This value is represented in the dynamic curves by the distance between the "true" value of the descriptor (circle on the curve) and the nearest thresholds b [from *n>s* to *n<s*] and *a* [from *n<s* to *n>s]*. In the example given, the values obtained are shown in the table below:

|  | increase | decrease |
|---|---|---|
| $i'_2$: black area | --- | --- |
| $i'_3$: variegation | --- | **23%** |
| $i'_4$: red reflectance | **44%** | --- |
| $i'_5$: IR reflectance | --- | **45%** |
| $I'_6$: dimension | --- | **23%** |

As will be seen by comparing the data in the table with those contained in the chart in figure 11, the quantities differ. This clearly emerges in the case of dimension; although the distance that separates it from the zone of non-malignant lesions is equal to that of variegation (see figures 10c and f), its determinance is much lower. This behaviour is due to the fact that the value range acquired by variegation for a classification of melanoma (from *b* to 100) is lower than that of dimension (from b to 100).

Classification of lesions into 4 or more different categories

To complete the explanation given so far, dynamic analysis allows lesions to be assigned to two or more classes. An example of the criteria for classification of lesions into 4 different categories (in this specific case, non-melanoma, doubtful, suspect and melanoma) is set out below.

A lesion originally classified as non-melanoma by the neural network is classified as *doubtful* if the risk of increase or decrease in at least two descriptors is greater than 0.8. As these descriptors are close to the threshold, despite the classification of non-malignancy supplied by the neural network it is preferable to take a cautious attitude and assign the lesion to a category other than "non-melanoma". Similarly, a lesion originally classified as melanoma by the neural network is assigned to the category of *suspect* lesions if, when the dynamic curves are observed, the distance between the point corresponding to the lesion and the threshold is less than 0.2 for at least two descriptors.

Example: according to this classification criterion, the non-malignant lesion referred to above would be classified as doubtful, because $p_{inc}(1) = 0.91$, $p_{inc}(3) = 0.85$, and $p_{inc}(6) = 0.83$ (see figures 7a, c and f).

Comparison between new lesions and data archive

As in the case of any classification model, the results supplied by the neural network depend on the cases used to teach the network. The fewer the lesions belonging to the teaching cases (stored in the data archive), the greater the probability that a new lesion will not be "similar" to any of the previously acquired lesions.

The *similarity s* between a lesion *m* present in the data archive (characterised by the set of descriptors $\{i_1, i_2, ..., i_6\}$) and a new lesion ($\{i^*_1, ...,i^*_6\}$) can be quantified with the equation

$$s(m) = \sqrt{\sum_{d=1}^{6}(i'_d(m) - i^{\bullet}_d)^2}$$

Of all the lesions in the data archive, the lesion *m* "most similar" to the one acquired is characterised by the lowest s value, which will be indicated as $s^*$

$$s^* = \min_m [s(m)]$$

At this stage a quantity $\alpha$, called the *lesion atypicality index*, can be defined with the equation

$$\alpha = e^{s^* - \frac{\sigma}{N}}$$

wherein $\sigma$ is a pre-set threshold value and $N$ corresponds to the number of lesions present in the data archive. The smaller the value of $\alpha$, the greater the similarity between lesion $m$ and the new lesion. If $\alpha > 1$, the message "Warning, no similar lesions in data archive" will be displayed at the end of processing of the lesion, and the classification given must be taken with a greater degree of caution. The value shown by the atypicality index is still of clinical interest, however, precisely because it characterises the atypicality of the lesion in question.

Study of the similarity of lesions can lead to a different classification criterion or a refinement of the criterion previously described. In the former case it may be decided, for example, that if there is at least one melanoma among the ten lesions "most similar" to the new lesion acquired, the new lesion should be classified as a melanoma regardless of the response given by the neural network.

If it is wished to refine the classification obtained with the neural networks, the two classification criteria can be combined as shown in the table below.

| Response of neural network | Similarity to melanoma | **CLASSIFICATION** |
|---|---|---|
| Probable non-melanoma | no | Probable non-melanoma |
| Probable non-melanoma | yes | Doubtful |
| Doubtful | no | Doubtful |
| Doubtful | yes | Suspect |
| Suspect | no | Suspect |
| Suspect | yes | Probable melanoma |
| Probable melanoma | no | Probable melanoma |
| Probable melanoma | yes | Probable melanoma |

**Claims**

1. Apparatus for the characterisation of pigmented skin lesions, **characterised in that** it comprises:

   - means designed to acquire images of the lesion, filmed with lighting at different wavelengths;
   - means designed to segment and parameterise each of said images;
   - means designed to extrapolate a data set from said images;
   - means designed to generate a neural network;
   - means designed to process the data relating to a set of known cases and define a threshold value on the basis of said processing;
   - means designed to input said data extrapolated from said images into the neural network;
   - means designed to compare the results processed by said neural network with said threshold value;
   - means designed to vary the weighting of each parameter supplied to the neural network on the basis of said results.

2. Apparatus for the characterisation of pigmented skin lesions as claimed in claim 1, **characterised in that** it comprises:

   - means designed to process images filmed with light at different wavelengths to extract the descriptors of the lesion;
   - means designed to reduce the number of said descriptors by factorial analysis in order to select a limited number of variables which retain over 85%, and preferably at least 95% of the variance.

3.  Apparatus for the characterisation of pigmented skin lesions as claimed in claim 1, **characterised in that** it comprises means designed to store an archive containing the values of the descriptors relating to all the images stored, and means designed to normalise the values of said descriptors by means of a function of the following type:

$$i_n'(m) = \frac{i_n(m)}{i_{n,\max} - i_{n,\min}} + \frac{i_{n,\min}}{i_{n,\min} - i_{n,\max}}$$

wherein $i_{n,min}$ and $i_{n,max}$ are the minimum and maximum value respectively of each descriptor n, among all the values of the lesions previously acquired.

4.  Apparatus for the characterisation of pigmented skin lesions as claimed in claim 2, **characterised in that** said means designed to film images of the lesion consist of a video camera associated with an illuminator comprising a light source and a rotating mirror with diffraction grid and means designed to control the rotations of said mirror to vary the wavelength of the light, said video camera being fitted with sensors designed to film a black and white image which are sensitive to wavelengths of light between 480 and 1000 nanometers, and sensors designed to film a colour image of the lesion.

5.  Apparatus for the characterisation of pigmented skin lesions as claimed in claims 3 and 4, **characterised in that** it comprises means designed to obtain from said images, for each lesion, at least the dimensions, variegation, reflectance in the visible and infra-red light zones, the presence of dark patches and the ratio between the area of the dark patches and the rest of the lesion.

6.  Apparatus for the characterisation of pigmented skin lesions as claimed in each of the preceding claims, **characterised in that** it comprises:

    • means designed to select a lesion;
    • means designed to vary the value of each descriptor by assigning to it a set of values which fall within a predetermined interval, the values of all the other descriptors being maintained unchanged;
    • means designed to input said values into the neural network to generate an output value, and means designed to construct a curve with said output values;
    • means designed to display a point on said curve corresponding to the value actually measured by the descriptor represented in said curve; and
    • means designed to display on a graph the intersections of said curve with a line representing said threshold value.

7.  Apparatus for the characterisation of pigmented skin lesions as claimed in claim 6, **characterised in that** it comprises means designed to show geometrical parameters, such as the distance between said threshold value and said point and/or the area under the curve in the zone between said threshold and said point, on one of the axes of the graph, and to derive from said measurement a value indicating the influence of a variation in one of the descriptors on the classification of a lesion.

Figure 1

Figure 2

Figure 3

Figure 4

$i_1 = 0.3967 \; ; \; i'_1 = 0.2540$

$i_2 = 0.7591 \; ; \; i'_2 = 0.7735$

$i_3 = 0.5452 \; ; \; i'_3 = 0.5876$

$i_4 = 0.0536 \; ; \; i'_4 = 0.5835$

$i_5 = 0.4446 \; ; \; i'_5 = 0.4983$

$i_6 = 32.2794 \; ; \; i'_6 = 0.1976$

Input descriptors

HS

$n = 1.000$

$s = 0.776$

hidden layer

**Figure 5**

**Figure 6**

Fig. 7a

Fig. 7b

Fig. 7c

Fig. 7d

Fig. 7e

Fig. 7f

**Figure 8**

**Figure 9**

**Fig. 10a**

**Fig. 10b**

**Fig. 10c**

**Fig. 10d**

**Fig. 10e**

**Fig. 10f**

**Figure 11**

**European Patent Office**

# EUROPEAN SEARCH REPORT

Application Number

EP 04 00 6186

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | US 6 208 749 B1 (GUTKOWICZ-KRUSIN DINA ET AL) 27 March 2001 (2001-03-27)<br>* abstract *<br>* column 3, line 30 - column 4, line 53 *<br>* column 6, line 58-68 *<br>* column 12, line 25-55 *<br>* column 15, line 55 - column 21, line 55 * | 1,2,4,5 | A61B5/103 |
| A | WO 01/35827 A (POLARTECHNICS LTD ;BATRAC ANDREW (AU); MENZIES SCOTT (AU); TALBOT) 25 May 2001 (2001-05-25)<br>* page 52, line 30 - page 58, line 34 * | 1-7 | |
| A | GANSTER H ET AL: "AUTOMATED MELANOMA RECOGNITION"<br>IEEE TRANSACTIONS ON MEDICAL IMAGING, IEEE INC. NEW YORK, US,<br>vol. 20, no. 3, March 2001 (2001-03),<br>pages 233-239, XP001096769<br>ISSN: 0278-0062<br>* the whole document * | 1-7 | TECHNICAL FIELDS SEARCHED (Int.Cl.7)<br><br>A61B |
| A | US 5 016 173 A (TEARNEY GUILLERMO J ET AL) 14 May 1991 (1991-05-14)<br>* the whole document * | 1-7 | |

The present search report has been drawn up for all claims

| Place of search | Date of completion of the search | Examiner |
|---|---|---|
| MUNICH | 21 July 2004 | Dhervé, G |

EPO FORM 1503 03.82 (P04C01)

**ANNEX TO THE EUROPEAN SEARCH REPORT
ON EUROPEAN PATENT APPLICATION NO.**

EP 04 00 6186

This annex lists the patent family members relating to the patent documents cited in the above-mentioned European search report.
The members are as contained in the European Patent Office EDP file on
The European Patent Office is in no way liable for these particulars which are merely given for the purpose of information.

21-07-2004

| Patent document cited in search report | | Publication date | Patent family member(s) | | Publication date |
|---|---|---|---|---|---|
| US 6208749 | B1 | 27-03-2001 | US | 6307957 B1 | 23-10-2001 |
| | | | AU | 740638 B2 | 08-11-2001 |
| | | | AU | 6340898 A | 18-09-1998 |
| | | | EP | 1011446 A1 | 28-06-2000 |
| | | | US | 6081612 A | 27-06-2000 |
| | | | WO | 9837811 A1 | 03-09-1998 |
| | | | US | 2002048170 A1 | 25-04-2002 |
| WO 0135827 | A | 25-05-2001 | WO | 0135827 A1 | 25-05-2001 |
| | | | AU | 1257701 A | 30-05-2001 |
| | | | EP | 1229835 A1 | 14-08-2002 |
| | | | JP | 2003513735 T | 15-04-2003 |
| US 5016173 | A | 14-05-1991 | AU | 5524990 A | 16-11-1990 |
| | | | WO | 9013091 A1 | 01-11-1990 |
| | | | US | 5836872 A | 17-11-1998 |
| | | | US | 5241468 A | 31-08-1993 |